# EUROPEAN PATENT SPECIFICATION

(11) **EP 1 507 867 B1**
(45) Date of publication and mention of the grant of the patent: **03.08.2011**
(21) Application number: 03734636.8
(22) Date of filing: 03.02.2003
(51) Int. Cl.: C12P 33/16

(54) **PROCESS FOR FERMENTATION OF PHYTOSTEROLS TO ANDROSTADIENEDIONE**
VERFAHREN FÜR DIE FERMENTATION VON PHYTOSTEROLEN ZU ANDROSTADIENDION
PROCEDE DE FERMENTATION DE PHYTOSTEROLS EN ANDROSTADIENEDIONE

(30) Priority: 01.02.2002 US 66395
(43) Date of publication of application: 23.02.2005
(73) Proprietor: N.V. Organon, 5349 AB Oss (NL)
(72) Inventor: KUTNEY, James, P., Vancouver, British Columbia V6G 3H2 (CA); HERRINGTON, Edward, J., Vancouver, British Columbia V6R 2K9 (CA); SPASSOV, Grigor, Vancouver, British, Columbia V6G E1E (CA)
(74) Representative: Kraak, Hajo
(86) International application number: PCT/CA2003/000131
(87) International publication number: WO 2003/064674

(56) References cited:
- WO-A-86/05813
- WO-A-99/49075
- US-A- 5 516 649

## Description

### FIELD OF THE INVENTION

This invention relates in general to fermentation processes, and in particular to the bio-conversion of phytosterol compositions to androstenedione and/or androstadienedione.

### BACKGROUND OF THE INVENTION

At present there are two main routes for the industrial production of steroid drugs. One is the diosgenin route and the other is the microbiological transformation route. Diosgenin is a spiroketal type steroidal a glycone isolated f rom a C omposite plant *(Dioscorea sp.).* In the late 1940's and early 1950's the route was developed using mainly chemical reactions:

Diosgenin Route for Production of Steroid Compounds.

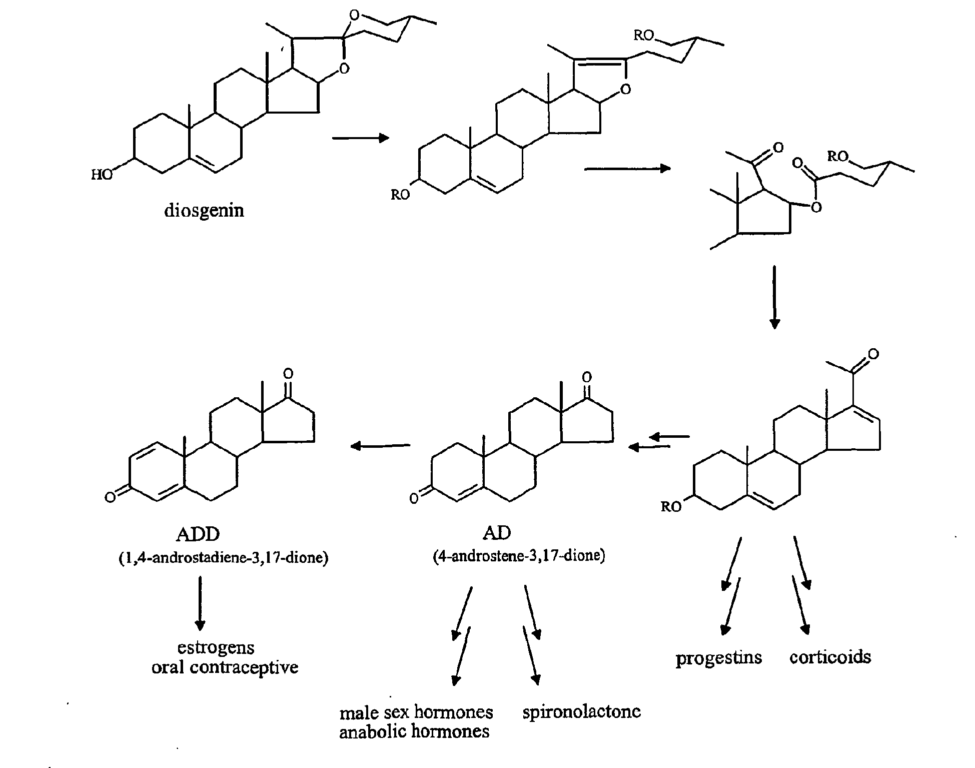

Although this route remained important for the commercial production of steroids, alternative methods came under consideration in the 1970's. The price of diosgenin was significantly increased particularly by the Mexican government and due, in part, to decreased content of diosgenin in the dry plant mass, and increased labor cost, etc. Therefore, an alternative methodology was required for future AD production.

It is well known from extensive research in the pharmaceutical industry in the 1960's and 70's that numerous microorganisms (*e.g*., those of the genera *Arthrobacter, Brevibacterium, Microbacterium, Protaminobacter, Bacillus, Nocardia, Streptomyces* and, in particular, *Mycobacterium)* possess the natural ability of degrading naturally occurring 3β-hydroxy-Δ⁵-sterols (such as cholesterol, β sitosterol or α-sitosterol) or a mixture of sterols (as shown below) to carbon dioxide and water, and that 4-androstene-3,17-dione (androstenedione) and 1,4-androstadiene-3,17-dione (androstadienedione) are formed as intermediates during the degradation.

Phytosterol Compounds Used as Substrates for Microbial Biotransformation.

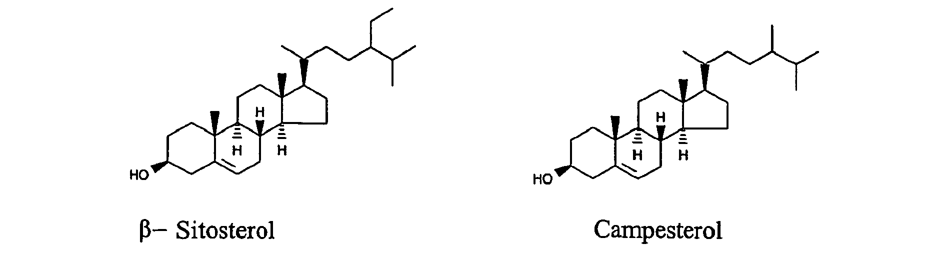

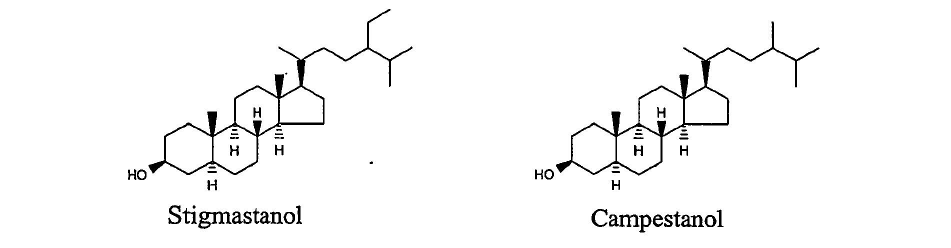

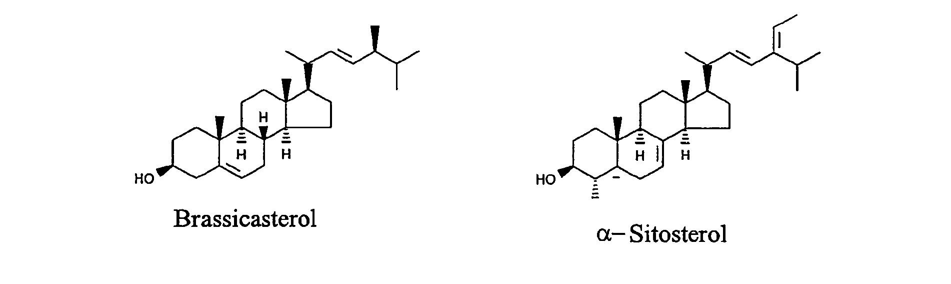

One of the present commercial sources of phytosterols are vegetable-derived oils. The microbial conversion of these available sterols to AD and ADD provides a highly important route to the steroid drug industry.

ADD is an essential raw material in the manufacture of various synthetic steroids including anti-inflammatory agents, oral contraceptives, synthetic adrenal steroids, anabolic agents (growth stimulating), and other synthetic steroids.

In general, the known fermentation process involves the propagation of a mutant of *Mycobacterium* in an appropriate nutrient medium, transfer of the culture to a bioreactor containing the phytosterols, and then allowing the biotranformation to AD and/or ADD over a period of approximately 120 hours. Harvesting of the fermentation broth, extraction of the latter with an organic solvent, and subsequent crystallization in an organic solvent, generally provides the products AD and/or ADD as white crystalline powders. Pertinent references that discuss the known process and summarize the earlier studies are as follows:
S. Kraychy, and R.D. Muir, U.S. Patent No. 3.684.657 (1972). W.J. Marsheck, S. Kraychy and R.D. Muir, Appl. Microbiol., 23, 72 (1972).
A.H. Conner, M. Nagaoka, J.W. Rowe and D. Perlman, Appl. and Environ, Microbiol., 32, 310 (1976).
K. Kleslich, J. Basic Microbiol., 25, 461 (1985). Concerning the patent application WO 86/05813, granted as European patent EP 0 217 840 B1, it refers to the production of 1-methyl-1,4-androstadiene-3,17-dione by fermentation of Metenolone (17β-hydroxy-1β-methyl-5α-androst-1-en-3-one) which is different from the compound used in the process of the present invention.

Furthermore, US5,516,649 discloses a process for the production of androst-4-one-3,17-dione (AD) and androsta-1,4-diene-3,17-dione (ADD) from ergosterol in the presence of Mycobacterium spec. NRRL B-3805.

GB 792,803 discloses in Example 1 the conversion of AD Into ADD in the presence of Fusarium solani.

One of the problems associated with the known phytosterol bio-conversion process (which problem plagues the entire steroid industry) involves the poor solubility of the substrate, in this case a phytosterol composition, in the aqueous nutrient medium. Inadequate solubility dictates the presence of only relatively low concentrations of substrate in the nutrient medium, resulting in poor contact with the micro-organism and generally leading to low yields of end products. Long fermentation times are also typically needed to achieve any satisfactory degree of big-conversion. To afford an alternative route to AD and ADD in which the issue of substrate solubility was addressed, the present applicants demonstrated that the phytosterols (ß □-sitosterol, campesterol and stigmastanol) obtained from "soap", available in multi-tonne quantities from the forest industry, could be solubilized and biotransformed quantitatively by Mycobacterium mutant to ADD ( WO 99149075).

Another problem associated with the known phytosterol big-conversion process is that the end-product of the big-conversion from phytosterols (or compositions of phytosterols) typically contains significant amounts of both AD and ADD. Given the similar chemical; structure of AD and ADD, it is difficult and expensive to subsequently separate these two steroid products from one another.

Accordingly in order to produce ADD, the product of the initial fermentation (AD) must be extracted, purified and then subjected to a second biotransformation in order to produce ADD. Naturally, this process is expensive and time-consuming as it involves the interruption of the AD fermentation, and the removal and purification of AD prior to the second biotransformation.

It is an object of the present invention to obviate or mitigate the above disadvantages.

### SUMMARY OF THE INVENTION

The present invention provides, in one aspect, a process of fermenting a phytosterol composition to produce androstenedione (androst-4-ene-3,17-dione or "AD") and subsequently androstadienedione (androsta-1,4-diene-3,17-dione or "ADD")) which comprises:
propagating a microbial culture of the genus *Mycobacterium* in a first nutrient medium; placing the microbial culture and the phytosterol composition in a bioreactor for a sufficient time to transform the composition substantially to androstenedione (the "AD solution"); propagating a fungal culture of the genus *Fusarium* in a second nutrient medium; and then injecting one or more of 1) *Fusarium sp;* or 2) the fungal culture medium comprising *Fusarium sp.* into the bioreactor for a sufficient time to transform the AD solution to androstadienedione.

The present invention further comprises a process of preparing andrestadienedione from androstenedione which comprises fermenting AD in a bioreactor in the presence of 1) *Fusarium sp;* or 2) a *Fusarium sp* fungal culture medium for a sufficient time to transform the AD into ADD.

The present invention further comprises compositions of ADD prepared by the aforementioned processes.

Importantly, and a significant advantage of the process of the present invention, is that the biotransformation of AD to ADD by *Fusarium sp* occurs in the same bioreactor as the prior bacterial transformation i.e. it is the same one in which AD is produced from the phytosterol composition. In other words, the AD produced from the initial fermentation step is not removed from the bioreactor (as is known and practised in the art) but rather, the bioreactor is inoculated (as described in more detail below) with either 1) the fungal culture, or 2) the fungal culture medium in order to transform the AD solution into ADD. It has been found that, surprisingly, *Fusarium sp* and in particular the preferred strain as disclosed hereinbelow retains its capacity to transform AD to ADD despite the presence of the residual bacterial culture

Accordingly, the process of the present invention does not involve the interruption of the Initial AD fermentation, or the removal and purification of AD prior to the second biotransformation. Significant time, energy and materials are saved.

The present invention also provides a novel microorganism of the genus *Fusarium,* hereinafter referred to as PTA-3947 or FMI 33, which was deposited at ATCC as *Fusarium solani* FMI 33 on December 21, 2001.

### DESCRIPTION OF THE DRAWINGS

The present invention is illustrated by the following non-limiting drawings in which:
Figure 1 is a schematic showing the m icrobial conversion of phytosterols to ADD in accordance with the present invention; and
Figure 2 is a flow diagram showing the preferred procedure for biotransformation of AD to ADD by *Fusarium solani in* accordance with the present invention.

### DETAILED DESCRIPTION OF THE INVENTION

The following detailed description is provided to aid those skilled in the art in practising the present invention. Modifications and variations to the embodiments discussed herein may be made by those with ordinary skill in the art .

In a first step of the present invention, a microbial culture of the genus *Mycobacterium* Is propagated in a nutrient medium. There are many suitable cultivation media and conditions known and available In the art and the present invention is not intended to be limited to any one. Nonetheless, in a preferred form, the *Mycobacteria sp.* are grown in a seed medium comprising refiner's molasses, sodium nitrate, ammonium phosphate and glucose. The contents of US Patent No 6,071,714 to Kutney et al., describes appropriate culture media

Generally, the nutrient media required for cultivation of the Mycobacteria sp and the Fusarium sp should contain assimilable nitrogen and carbon and an adequate supply of sterile air maintained therein, for example by exposing a large surface of the media to air or passing it through the media in quantities sufficient to support submerged growth.

Suitable nitrogen sources are those normally employed for the purpose, including soybean meal, cottonseed meal, pancreatic digest of casein, fish meal, corn steep liquor, meat extract, protein, autolyzed brewer's yeast with milk solids, peptone, yeast extract, casein hydrolysate, nitrate and/or ammonium compounds. Most of these may also be used as the carbon source. Other carbon containing substances include carbohydrates such as glycerol, glucose, fructose, sucrose, lactose, maltose, inositol, dextrin, molasses, starch and whey. Combinations of these carbon and nitrogen sources may be used advantageously.

Phosphate, magnesium and/or ferrous ions may be incorporated as growth promoting factors if desired; buffers may be added to assure that growth is initiated as substantially neutral pH. Anti-foaming agents may be beneficial. Where isolated cells or enzymes are used to induce fermentation rather than the intact and growing microorganism, nutrients need not be present; but in either event the medium is usually mostly aqueous.

In a second step of the present invention, the substrate (a phytosterol composition), dissolved in a suitable solvent or in emulsified form, and the microbial culture are added to a bioreactor. Fermentation is allowed to proceed until maximum substrate conversion (phytosterols to AD solution) is achieved.

Any emulsifying agent may be used as long as it facilitates mixing of the phytosterol substrate in the solvent thereby optimizing availability of the substrate to the microbial culture. Examples include, but are not limited to ethyleneoxy adducts or fatty acid esters of polyglycols and those commercially available under the names Tegin™, Tween™ and Span™.

In the alternative, solubilizing agents may be used to allow the phytosterol composition to be dissolved to form a clear solution, thereby affording excellent contact with the micro-organism utilized in the bio-conversion. These selected suitable solubilizing agents, which include members of the glycol family and members of the silicone family, allow high concentrations of phytosterols to be dissolved in the nutrient medium. This provides for excellent contact with the micro-organism, reduces fermentation times, and affords relatively high yields of end-products. Prior-known solubilizing agents such as sunflower oil are preferred.

In a third step of the present invention, a fungal culture of the genus *Fusarium* is propagated in a second nutrient medium. As above, there are many suitable cultivation media and conditions known and available in the art and the present invention is not intended to be limited to any one. Nonetheless, in a preferred form, the *Fusarium sp.* are grown in a seed medium comprising corn steep liquor and glucose.

In a fourth step of the present invention, the bioreactor comprising the AD solution is injected with one or more of: 1) the *Fusarium,* or 2) the fungal culture medium (comprising fungal enzymes) in order to transform the AD solution into ADD. In a preferred embodiment, the bioreactor is sterilized (for example by heating at around 120° C) prior to this injection to kill the bacteria.

The simplest and most economical way of proceeding with this "injection" step is to inoculate the sterilized bioreactor with some of the fungal culture medium. Alternatively, however, it is possible to separate the fungal cells from the culture medium by, for example, centrifugation and just inject these cells. Moreover, the cells can be ruptured, ultrasonically or otherwise, to facilitate access to the enzymes present, which can be isolated by filtration or extracted with a solvent such as acetone and water and then injected into the bioreactor instead of the medium or fungal cells.

The optimal substrate concentration, substrate addition time and fermentation period in each of the two "phases" (phytosterols to AD; AD to ADD) depends on the type of microorganism used and the exact fermentation conditions. The precise conditions could readily be determined without undue experimentation by anyone skilled in this field.

In a most preferred form of the present invention, the bioconversion of the phytosterol composition to AD is achieved by the use of *Mycobacterium* MB 3683. In a most preferred form of the present invention, the bioconversion of AD to ADD is achieved by the use of either *Fusarium solani* or *Fusarium oxysporum.*

Within one embodiment of the present invention, mutation of *F. solani,* using nitrosoguanidine has resulted in the production of a novel mutant which is especially effective in transforming AD to ADD in the same reaction vessel as the transformation of the steroids to AD i.e.without the necessity of separating AD from the first fermentation step. This mutant microorganism has been given the patent deposit designation PTA-3947 by the American Type Culture Collection ("ATCC"), 10801 University Blvd., Manassas VA, USA, 20110-2209, where it has been deposited in a permanent collection. A subculture of this microorganism is available upon request thereto, however, it should be understood that such availability does not constitute a license to practise the subject invention.

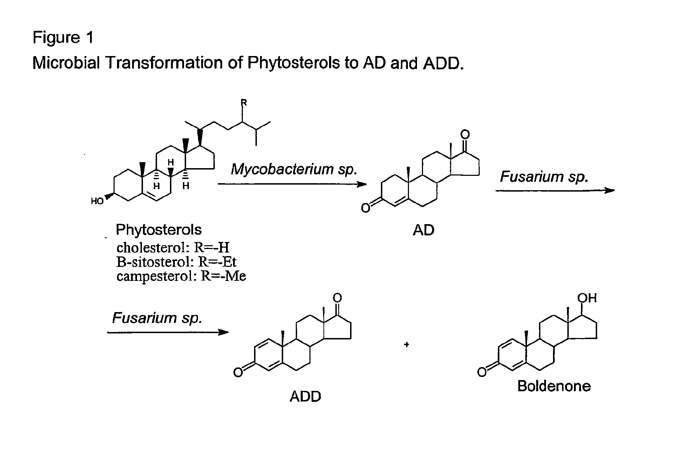

Figure 1 outlines the microbial and fungal fermentation steps of one embodiment of the present invention wherein phytosterols are transformed by *Mycobacterium sp.* to AD and subsequently the AD is transformed by *Fusarium sp* to ADD and boldenone.

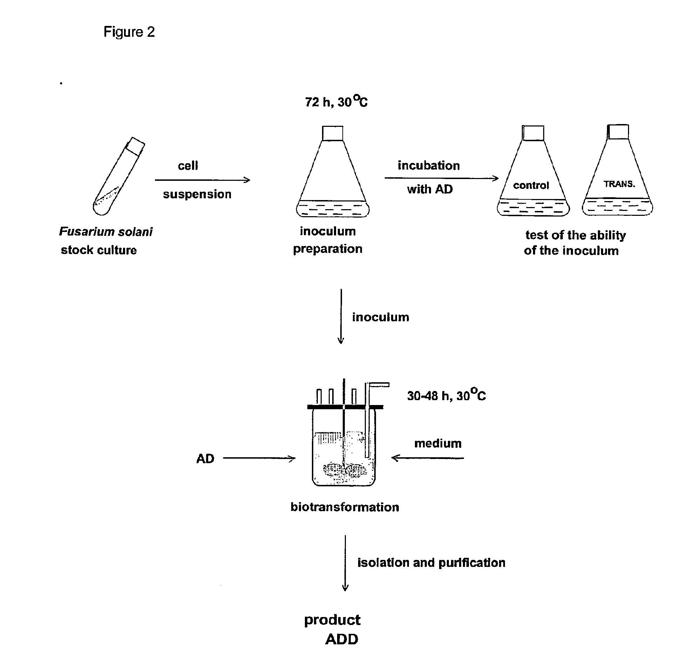

Figure 2 illustrates a further embodiment of the present invention wherein ADD is prepared directly from an AD source. A *Fusarium sp.* culture is propagated and the inoculum added to a vessel comprising AD under fermentation conditions. Thereafter ADD is isolated and purified.

### Phytosterols/Phytostanols

As used herein, the term "phytosterol composition" includes all sterols without limitation, for example: sitosterol, campesterol, stigmasterol, brassicasterol (including dihydrobrassicasterol), desmosterol, chalinosterol, poriferasterol, clionasterol, ergosterol, coprosterol, codisterol, isofucosterol, fucosterol, clerosterol, nervisterol, lathosterol, stellasterol, spinasterol, chondrillasterol, peposterol, avenasterol, isoavenasterol, fecosterol, pollinastasterol, cholesterol and all natural or synthesized forms and derivatives thereof, including isomers. and includes all hydrogenated counterparts ("phytostanols"). Phytostanols includes all saturated or hydrogenated phytosterols and all natural or synthesized forms and derivatives thereof, including isomers. It is also to be understood that, when in doubt throughout the specification, the term "phytosterol composition" may encompass both phytosterols and phytostanols.

The sterols and stanols for use in the biotransformation of the present invention may be procured from a variety of natural sources. For example, they may be obtained from the processing of plant oils (including aquatic plants) such as corn oil and other vegetable oils, wheat germ oil, soy extract, rice extract, rice bran, rapeseed oil, sunflower oil, sesame oil and fish (and other marine-source) oils. They may also be derived from fungi, for example ergosterol. Accordingly, the present invention is not to be limited to any one source of sterols. US Patent Serial No. 4,420,427 teaches the preparation of sterols from vegetable oil sludge using solvents such as methanol. Alternatively, phytosterols and phytostanols may be obtained from tall oil pitch or pulping soap, by-products of forestry practises as described in US Patent Serial No.5,770,749.

During the fermentation process, it is preferred that the temperature be maintained in the range of about 30-37°C, more preferably from 25-35° C. Monitoring of the pH during the fermentation reveals that the pH can vary in the range of approximately 7.0 in the initial period to approximately 4.7 at harvest time. Upon completion of the fermentation or transformation process, the ADD is recovered by means well known and practised in the art.

The following examples represent laboratory trials. However, each can be extrapolated to industrial-scale application using known industrial techniques to implement the invention as described herein.

### EXAMPLE 1 BIOTRANSFORMATION OF PHYTOSTEROLS TO ADD WITH MYCOBACTERIUM SP. AND FUSARIUM SOLANI

### Biotransformation with Mycobacterium sp. (Stage 1)

### Preparation of Seed Culture of Mycobacterium sp.

Inoculum for biotransformation is prepared in liquid culture. The Erlenmeyer flasks are inoculated from an agar slant (grown in a glass test tube). For working volumes up to 40 L, 1 L Erlenmeyer flasks with 200 mL seed culture medium each were used, while for 50 L and larger volumes 2 L Erlenmeyer flasks with 400 mL medium each were utilized. The inoculation is performed under sterile conditions (in a Biohazard Hood). Sterile water (5 mL) is pipetted onto the agar slant. The bacterial culture is gently scraped with the end of the pipette. Then the suspended culture is pipetted and released into the Erlenmeyer flasks. The bacteria is propagated on a rotary shaker (200 rpm, 1° throw, 30°C ambient temperature). After 72 hours of incubation the seed culture is ready for transfer to the bioreactor. Prolonged storage of the seed culture before inoculation is not recommended.

A summary of the Seed Culture protocol is given below:

| medium: | seed culture medium (per L) |
|---|---|
| | 54 mL Refiners molasses (BC sugar) |
| | 5.4 g NaNO₃ |
| | 0.6 g NH₄H₂PO₄ |
| | 6.0 g glucose - |
| | adjust to pH 7.0 |
| medium volume: | 200 mL/flask |
| inoculum volume: | 3ml cells suspension from agar slant per 200 mL new medium |
| vessel: | 1000 mL Erlenmeyer flask |
| agitation: | shaker (1" throw), 200 rpm |
| temperature: | 30°C |
| time of growth: | 72 h incubation |

| | |
|---|---|
| Comment: Good culture growth is indicated if a yellow coloured cell sediment forms at the bottom. | |

### Preparation of Biotransformation Medium for 3 L Experiments with Mycobacterium sp.

This procedure of medium preparation is utilized only for 3 L working volume experiments. At a larger scale the procedure described below.

The composition of the biotransformation medium is as follows:

| medium: | biotransformation medium | |
|---|---|---|
| | (for 3 Liters of medium) | |
| | 162 mL sterilized molasses (BC Sugar) | (5.4% v/v) |
| | 16.2g NaNO₃ | (0.54%) |
| | 1.8gNH4H₂PO₄ | (0.06%) |
| substrate: | 30.0 g vegetable phytosterol | (1.0%) |
| emulsifier: | 480 mL sunflower oil | (16%) |

The necessary amount of Refiners molasses (162 mL) is mixed with water (300 mL) and sterilized (121°C for 20 min) in an Erlenmeyer flask one day before use.

In a flask or a beaker water (2.0 L) and the medium components (except the phytosterols and the sunflower oil) are mixed. The pH of the medium is adjusted to 8.1 with 1 M NaOH solution (40 g/L NaOH), the medium is transferred to the bioreactor and then heated to 60-70°C.

Phytosterols (30 g) are weighed in a beaker, sunflower oil (480 mL) is added and the mixture is stirred under heating on a hot plate until the oil becomes clear. This is usually reached at ~120°C. The clear yellow phytosterol solution in oil is poured into the already warmed liquid medium (60-70°C) in the bioreactor without stirring.

The fermentor is sterilized (121°C for 30 min) and cooled to 30°C.

The above procedure of medium preparation is utilized only for 3 L working volumes.

Comments: The biotransformation medium has 7 % dry substance (refractometrically measured).

### Preparation of Biotransformation Medium for Large Scale Experiments with Mycobacterium sp.

This procedure of medium preparation is utilized for 40 L and larger working volumes.

**Table 1. Composition of Vegetable Phytosterols according to manufacturer**

| # | Name | | Rt (min) | Amount (%) |
|---|---|---|---|---|
| | Mono C16 | * | 9.378 | 0.10 |
| | Mono C18 7 | * | 10.698 | 0.12 |
| | d-Tocopherol | ** | 11.291 | 0.18 |
| | γ-Tocopherol | ** | 11.900 | 0.12 |
| | Cholesterol | ***C | 12.625 | 0.29 |
| | α-Tocopherol | ** | 12.726 | 0.03 |
| | Brassicasterol | *** | 12.893 | 4.43 |
| | Campesterol | *** | 13.253 | 27.71 |
| | Campestanol | *** | 13.301 | 0.44 |
| | Stigmasterol | *** | 13.438 | 18.13 |
| | β-Sitosterol | *** | 13.765 | 44.88 |
| | Sitostanol | *** | 13.855 | 1.15 |
| | MAG (*) | | | 0.22 |
| | Tocopherols (**) | | | 0.33 |
| | Sterols (***) | | | 95.05 |
| | Stanols (***) | | | 1.59 |
| | Cholesterol (***^{C}) | | | 0.29 |
| | Phytosterols + Cholesterol | | | 96.93 |

The medium composition is as follows:

| medium: | biotransformation medium | |
|---|---|---|
| | (per Liter medium) | |
| | 54 mL sterilized molasses (BC Sugar) | (5.4% v/v) |
| | 5.4 g NaNO₃ | (0.54%) |
| | 0.6g NH₄H₂PO₄ | (0.06%) |
| substrate: | 10.0 g vegetable phytosterol | |
| (1.0%) | | |
| emulsifier: | 160 mL sunflower oil | (16%) |

The necessary volume of Refiners molasses (54 mUL medium) is mixed with water (100 mUL medium) and sterilized one day before use.

All components (including the necessary volume of water, but excluding the phytosterols and the sunflower oil) are mixed under stirring in the fermentor to obtain a clear solution. The necessary volume of water is calculated by substracting the volumes of molasses, oil and inoculum from the desired final volume. The pH of the medium is adjusted to 8.1 with aqueous NaOH solution (20%). The phytosterols and the sunflower oil are added to the medium and the fermentor is sterilized (121°C, 30-60 min).

Comments: The biotransformation medium has 7 % dry substance (refractometrically measured).

### Biotransformation with Mycobacterium in a Fermentor

The bioconversion experiments were performed in stainless steel fermentors with efficient bottom stirring. The main parameters of the utilized fermentors are shown in Table 2. The temperature of the medium in the fermentor is adjusted to 30°C and the inoculum is introduced under sterile conditions. The entire process is performed at 30°C. The pH and the sterility of the process are monitored from the beginning until the end of this stage. The monitoring of the substrate (phytosterols) and the product (AD) is initiated after 40 hours. Details on the analytical procedures (HPLC, GC) and results are given below. The pH of the biotransformation mixture usually varies within 1 pH unit and is not corrected during the process. The biotransformation is considered completed when maximal product (AD) concentration in the foam layer is achieved or when the substrate (phytosterol) concentration reaches 1/20-th of its initial concentration.

**Table 2. Parameters of Phytosterol Fermentation with Mycobacterium sp.**

| Parameter | Value | | | | |
|---|---|---|---|---|---|
| Total volume (L) | 10 | 40 | 100 | 1000 | 5000 |
| Working volume (L) | 3 | 30 | 50 | 500 | 2500 |
| Inoculum volume (L) | 0.4 | 3.0 | 2.4 | 50 | 500 |
| Inoculum age (hrs) | 72 | 72 | 72 | 48-70 | 48-70 |
| Aearation (L/L/min) | 0.2 | 0.2 | 1.0 | 1.0 | 1.0 |
| Agitation (rpm) | 300 | 300 | 200 | 200 | 200 |

| | | | | | |
|---|---|---|---|---|---|
| Important: A highly efficient stirring is required in order to obtain maximum contact with the micro-organism. In general, a "milk-shake" appearance characterizes a good emulsion | | | | | |

Note on morphology (as studied using a light microscope):
At every stage, from agar slant to final harvest, the morphology of the cells remained the same, that is, the cells had bipolar nuclei and were rod-shaped.

### Biotransformation with Fusarium so/ani (Stage 2)

### Preparation of Seed Culture of Fusarium solani

The fungal culture *Fusarium solani is* propagated in a liquid medium (10 g/L corn steep liquor, 10 g/L glucose, pH 6.5). E rienmeyer flasks (1 L volume, 0.2 L medium) a re inoculated from culture grown on oatmeal agar for 120 hours and at 30°C. The liquid medium culture is propagated on a rotary shaker (26°C, 120 rpm, 1° throw) for 72 hours.

*Fusarium* can also be sub-cultured from liquid to liquid medium for up to 4 - 5 generations or until the next culture becomes pink.

The shake flask inoculum could be stored at 4°C for up to 1 month without significant change in its activity.

### Preparation for Biotransformation with Fusarium solani

The second stage of the biotransformation is performed using the entire biotransformation broth from the first stage.

Upon completion of the transformation of phytosterols to AD the necessary components (10 g/L glucose, 10 g/L corn steep liquor) are added to the broth from stage 1 and pH is adjusted to 6.7 with aqueous HCl (4 %). The broth is sterilized (121°C, 30 min) and the temperature is brought to 30°C.

### Biotransformation of AD to ADD Utilizing Broth from Stage 1 and Fusarium solani

The bioconversion experiments were performed in stainless steel fermentors with efficient bottom stirring. The main parameters of the utilized fermentors are shown in Table 3. The temperature of the medium In the fermentor Is adjusted to 30°C and the Inoculum is introduced under sterile conditions.

**Table 3. Parameters of Phykosterol Fermentation with Fusarium solani.**

| Parameter | Value |
|---|---|
| Total volume (L) | 10 |
| Working volume (L) | 3.0 |
| Inoculum volume (L) | 0.2 |
| Inoculum age (hrs) | 72 |
| Aearation (L/L/min) | 0.2 |
| Agitation (rpm) | 200 |

The entire process Is performed at 30°C. The pH and the sterility of the process are monitored from the beginning until the end of this stage. The monitoring of the substrate (AD) and the product (ADD) is initiated after 15 hours. Details on the analytical procedures (HPLC, GC) are given in example 4. Results from the monitoring of the process are shown in example 4. The pH of the biotransformation mixture usually varies within 1 pH unit and is not corrected during the process. The biotransformation is considered completed when maximal product (ADD) concentration In the foam layer is achieved or when the substrate (AD) concentration reaches 1/20-th of its initial concentration.
The process is terminated by sterilization (121°C, 30 min).

### EXAMPLE 2 ONE STAGE BIOTRANSFORMATION OF AD TO ADD WITH FUSARIUM SOLANI

### Preparation of Seed Culture of Fusarium solani

The fungal culture *Fusarium solani* is propagated in a liquid medium (10 g/L corn steep liquor, 10 g/L glucose, pH 6.5). Erlenmeyer flasks (1 L volume, 0.2 L medium) are inoculated from culture grown on oatmeal agar for 120 hours and at 30°C. The liquid medium culture is propagated on a rotary shaker (26°C, 120 rpm, 1" throw) for 72 hours. *Fusarium* can also be sub-cultured from liquid to liquid medium for up to 4-5 generations or until the next culture becomes pink.
The shake flask inoculum could be stored at 4°C for up to 1 month without significant change in its activity.

### Preparation of Biotransformation Medium for Fusarium solani

This process is performed utilizing already isolated AD.

The composition of the biotransformation medium is as follows:

| medium: | biotransformation medium | |
|---|---|---|
| | (for 3 Liters of medium) | |
| | 30 g glucose | (10%) |
| | 30 mL corn steep liquor | (10% v/v) |
| substrate: | 30.0 g AD | (1.0%) |
| emulsifier: | 480 mL sunflower oil | (16%) |

In a flask or a beaker water (2.5 L) and the medium components (except the AD and the sunflower oil) are mixed. The pH of the medium is adjusted to 6.5 with 1 M NaOH solution (40 g/L NaOH), the medium is transferred to the bioreactor and then heated to 60-70°C.

AD (30 g) is weighed in a beaker, sunflower oil (480 mL) is added and the mixture is stirred under heating on a hot plate until the oil becomes clear. The clear yellow AD solution in oil is poured into the already warmed liquid medium (60-70°C) in the bioreactor without stirring.

The fermentor is sterilized (121°C for 30 min) and cooled to 30°C.

### Biotransformation of AD to ADD in a Stainless Steel Fermentor

The bioconversion experiments were performed in stainless steel fermentors with efficient bottom stirring. The main parameters of the utilized fermentors are shown in Table 4. The temperature of the medium in the fermentor is adjusted to 30°C and the inoculum is introduced under sterile conditions. The entire process is performed at 30°C. The pH and the sterility of the process are monitored from the beginning until the end of this stage. The monitoring of the substrate (AD) and the product (ADD) is initiated after 15 hours. Details on the analytical procedures (HPLC, GC) and results are given below. The pH of the biotransformation mixture usually varies within 1 pH unit and is not corrected during the process. The biotransformation is considered completed when maximal product (ADD) concentration in the foam layer is achieved or when the substrate (AD) concentration reaches 1/20-th of its initial concentration.

The process is terminated by sterilization (121°C, 30 min).

**Table 4. Parameters of Phytosterol Fermentation with Fusarium solani.**

| Parameter | Value |
|---|---|
| Total volume (L) | 10 |
| Working volume (L) | 3.0 |
| Inoculum volume (L) | 0.2 |
| Inoculum age (hrs) | 72 |
| Aearation (L/L/min) | 0.2 |
| Agitation (rpm) | 200 |

### EXAMPLE 3 EXTRACTION OF THE FERMENTATION BROTH

The fermentation broth is harvested into a separatory funnel. Ethyl acetate (6.0 L) is added to the broth and shaken for 2-3 min. After 15 min the mixture is separated into two layers (ethyl acetate upper layer and aqueous bottom layer). The ethyl acetate layer is removed and transferred to another vessel. The bottom aqueous layer is extracted with a second portion of ethyl acetate (4.0 L), then the upper (ethyl acetate) layer is removed and combined with the first ethyl acetate extract. The combined ethyl acetate extracts are concentrated on a rotary evaporator to give a red thick oil (~450 ml) which is used for a further purification.

Each ethyl acetate extract and the aqueous residue are analyzed for steroids by HPLC. The results for two stage process age shown in Table 5 and the results for one stage process are shown in Table 6.

### Partitioning of ADD from the Oil

The oily residue (~450 ml) is mixed well with hexanes (1.25 L). The oil-hexanes solution is extracted with MeOH (2.0 L × 2). The two methanol extracts are combined and evaporated until dry to give a beige oily residue. The residue is vacuum dried. Each of the hexanes and methanol layers is analyzed by HPLC to control t he completion of the partitioning.

### A) Crystallization of ADD from the Two Stage Fermentation of Phytosterols to ADD First Crystallization

In some experiments the residue after the evaporation of methanol is vacuum dried. If so, this residue (-40-50 g, ADD content 17-18 g, purity 30-40 % by HPLC) is dissolved in ethyl acetate (100 ml).

If the residue after the evaporation of methanol was not vacuum dried then this residue is dissolved in ethyl acetate (250 ml), dried over Na₂SO₄ and concentrated to a smaller volume (100 ml).

Charcoal (4.0 g) is added and the mixture is refluxed for 1 hr. After cooling, the mixture is filtered on a sintered glass funnel and the filter is washed with ethyl acetate. Alternatively, the solution can be filtered through a Celite pad and washed with ethyl acetate. The filtrate is concentrated on a rotary evaporator until dry. Hexanes (200 ml) are added, the mixture is stirred under reflux for 20 min, cooled to room temperature and left for 30 min to separate. The hexanes upper layer is transferred to another vessel and allowed to stand overnight for crystallization.. The ADD crystals from the hexanes solution are filtered, washed with hexanes/EtOAc (3/1) and dried under the reduced pressure to give white ADD crystals (0.23 g). The sticky oil is dissolved in ethyl acetate (8ml) and stirred under heating for 10 min. The solution is cooled to room temperature and allowed to stand overnight. The ADD crystals are filtered, washed with hexanes/EtOAc (3/1) and dried under the reduced pressure to give white ADD crystals (2.51 g).. The crystals and the mother liquor are analyzed by HPLC.

### Second Crystallization

The mother liquid left after the first crystallization is concentrated on a rotary evaporator. Ethyl acetate (4 ml) is added, the mixture is stirred under reflux for 10 min, cooled to room temperature and left overnight for crystallization. The ADD crystals are filtered, washed with hexanes/EtOAc (3/1) and dried under reduced pressure to give white ADD crystals (1.25 g). The product and the mother liquor are analyzed by HPLC.

**Table 5. Results from Extraction and Partitioning of the Product (ADD) from Two Stage Process.**

| Example | | | Extraction with EtOAc | | | Partitioning with MeOH | | |
|---|---|---|---|---|---|---|---|---|
| No. | Time (h) | Substr ate (g) | ADD (g) | AD (g) | Bold. (g) | ADD (g) | AD (g) | Bold. (g) |
| 444 | 90 / 46 | 30 | 6.86 | 0.80 | 1.08 | 6.29 | 0.08 | 0.95 |
| 445 | 91 / 42 | 30 | 6.71 | 0.90 | | 3.92 | 1.25 | 2.48 |
| 446 | 73 / 22 | 30 | 5.85 | 2.50 | 1.47 | 5.72 | 2.07 | 1.22 |
| 447 | 71 / 23 | 30 | 12.10 | 1.46 | 2.00 | 11.92 | 1.18 | 1.82 |

### Third Crystallization

The mother liquid left after the second crystallization is concentrated on a rotary evaporator. A mixture of ethyl acetate (2 ml) and hexanes (1 mL) is stirred under reflux for 10 min, cooled to room temperature and left overnight for crystallization. The ADD crystals are filtered, washed with hexanes/EtOAc (3/1) and then dried under reduced pressure to give white ADD crystals (0.16 g). The product and the mother liquor are analyzed by HPLC.

Total amount of crystallized ADD is 4.25 g.

### B) Isolation of ADD from the One Stage Fermentation of AD to ADD

### First Crystallization

If the residue after the evaporation of methanol was vacuum dried, then this residue (-40-50 g, ADD content 17-18 g, purity 30-40 % by HPLC) is dissolved in ethyl acetate (150 ml).

If the residue after the evaporation of methanol was not vacuum dried, then this residue is dissolved in ethyl acetate (250 ml), dried over Na₂SO₄ and concentrated to a smaller volume (150 ml).

Charcoal (4.0 g) is added and the mixture is refluxed for 1 hr. After cooling, the mixture is filtered on a sintered glass funnel and the filter is washed with ethyl acetate. Alternatively, the solution can be filtered through a Celite pad. The filtrate is concentrated on a rotary evaporator until dry, then the residue is dissolved in ethyl acetate (6 ml) under heating and cooled to room temperature for crystallization. After standing overnight the ADD crystals are filtered, washed with hexanes/EtOAc (3/1) and dried under reduced pressure to give white ADD crystals. The crystals and the mother liquor are analyzed by HPLC.

### Second Crystallization

The mother liquid left after the first crystallization is concentrated on a rotary evaporator. Hexanes (50 ml) are added, the mixture is stirred under reflux for 1 h, cooled to room temperature and left for 30 min to separate. The hexanes upper layer is transferred to another vessel and allowed to stand overnight for crystallization. The sticky oil is mixed with ethyl acetate (2ml), hexanes (1ml) and stirred under heating for 10 min. The solution is cooled to room temperature and allowed to stand overnight. The ADD crystals are filtered, washed with hexanes/EtOAc (3/1) and dried under the reduced pressure to give white ADD crystals. The hexanes layer also gave one portion of ADD crystals. The crystals and the mother liquor are analyzed by HPLC.

### Third Crystallization

The mother liquor from the second crystallization is concentrated on a rotary evaporator. The sticky oil is mixed with ethyl acetate (1ml), hexanes (3ml) and stirred under heating for 10 min. After cooling, the solution is allowed to stand overnight for crystallization. The ADD crystals are filtered, washed with hexanes/EtOAc (3/1) and dried under reduced pressure to give ADD crystals. The crystals and the mother liquor are analyzed by HPLC.

The results from ADD crystallization are shown in Table 6.

**Table 6. Results from Extraction, Partitioning and Crystallization of ADD from One Stage Process.**

| Example | | | Extraction with EtOAc | | | Extraction with MeOH | | | Crystallization | | | | |
|---|---|---|---|---|---|---|---|---|---|---|---|---|---|
| No | Time (h) | AD (g) | ADD (g) | AD (g) | Bold (g) | ADD (g) | AD (g) | Bold (g) | First ADD (g) | Sec ond hexanes ADD (g) | Sec ond EtO Ac ADD (g) | Third ADD (g) | Total ADD (g) |
| 454 | 24 | 26.52 | 18.83 | 1.37 | 1.07 | 18.72 | 1.41 | 1.03 | 13.62 | 0.85 | 1.44 | - | 15.91 |
| 455 | 39 | 26.52 | 17.20 | 1.20 | 1.22 | 15.26 | 1.78 | 1.08 | 7.43 | 4.31 | 2.30 | - | 14.04 |
| 456 | 48 | 26.52 | 19.64 | 0.54 | 1.00 | 17.32 | 0.00 | 1.76 | 10.02 | 4.46 | 2.03 | - | 16.51 |
| 457 | 46 | 26.52 | 18.27 | 0.58 | 1.58 | 19.20 | 0.58 | 1.56 | 4.01 | 8.32 | 1.96 | 1.87 | 16.16 |
| 458 | 42 | 26.52 | 19.75 | 0.00 | 1.00 | 19.75 | 0.00 | 1.00 | 7.83 | 3.72 | 2.14 | 0.89 | 14.58 |

### EXAMPLE 4 QUALITATIVE (TLC) AND QUANTITATIVE (HPLC AND GC) ANALYSIS

### Qualitative analysis by TLC

Broth samples (approximately 40 mL each) are taken from the bottom of the fermentor. The broth sample (0.5 mL) is extracted with EtOAc (0.5 mL). TLC analysis was carried out on silica gel pre-coated plates (60 F₂₅₄, thickness 0.2 mm) using 0.050 mL of the EtOAc extract. Mobile phase: h exanes/EtOAc = 3:1; c hloroform/acetone=3:1; or m ethylene chloride/acetone= 9:1 were utilized. After developing the plate, one of the following spray (or dip) reagents was used.

Cerrium reagent containing (for 100 mL): water (94 mL), cerium (IV) sulphate (1.0 g), phosphomolybdic acid hydrate (2.5 g), sulphuric add (6 ml). The components are dissolved in the order of listing. The reagent can be stored for more than 1 year.

Molibdate reagent containing (for 100 mL): water (90 mL), ammonium molibdate (10.85 g), sulphuric acid (6 ml). The components are dissolved in the order of listing. The reagent can be stored for more than 1 year.

The plate was then heated for 3 minutes at 150°C. The resulting blue spots signified the presence of the following compounds (hexanes/EtOAc = 3/1):
Rf = 0.75-.sunflower oil; Rf = 0.53-phytosterol;
Rf = 0.47-androstenedione(AD); and
Rf = 0.02-androstadienedione (ADD).

### Monitoring of AD, ADD and By-products by HPLC.

A sample (2 g foam or 2 mL liquid or 10 mg crystals) was diluted to 50.0 mL with methanol, mixed well and left for 10 min to precipitate the proteins and phytosterols. An aliquot of the solution was filtered (Millipore filter type HV 0.45 µm). HPLC analysis was performed using radialpack column (stationary phase C₁₈ 5µm, 4 x 100 mm, Waters). Mobile phase: water/ methanol = ratio 4:6 (isocratic). Flow 1 ml/min. Detection at 280 nm. Standard curve for AD (mg) = (8.39 Area + 2033) x 10⁻⁵; in which the area ranges from 0 to 30000.

Standard curve for ADD (g) = (7.43 Area + 72.22) x 10⁻⁴; in which the area ranges from 0 to 100000.

Standard curves were obtained from the results for pure samples of androstenedione (AD) and androstadienedione (ADD). The retention times of the products are as follows.

| Compound | Retention time (min) |
|---|---|
| AD | 8.73 |
| ADD | 13.20 |

### Monitoring the substrates and products by GC

The sample (50 mg crystals) is dissolved in EtOAc and dilluted to 50.0 mL in a volumetric flask. Alternatively, 2 mL biotransformation mixture is extracted with 50 mL EtOAc. The EtOAc solution is then analyzed for Phytosterols, AD and ADD by GC.

Conditions: SAC-5 column, oven temperature 278 °C, injector temperature 300 °C, detector FID.

The retention times of the products are as follows.

| Compound | Retention time (min) |
|---|---|
| AD | 8.06 |
| ADD | 9.21 |
| Brassicasterol | 20.53 |
| Campesterol | 23.20 |
| Campestanol | 23.66 |
| Stigmasterol | 24.70 |
| β-Sitosterol | 27.71 |
| Sitostanol (Stigmastanol) | 28.27 |

### Results from Biotransformation of Phytosterols and AD to ADD

**Table 7. Summary of the monitoring of Two Stage Biotransformation Process.**

| Time | Foam layer | | | Water layer | | |
|---|---|---|---|---|---|---|
| | ADD/AD | ADD | AD | ADD/AD | ADD | AD |
| (hrs) | (-) | (%) | (%) | (-) | (%) | (%) |
| First biotransformation (phytosterols to AD) | | | | | | |
| 0.0 | 0: 0 | 0.0 | 0.0 | 0: 0 | 0.0 | 0.0 |
| 87.5 | 11:89 | 4.0 | 30.4 | 25 : 75 | 0.6 | 1.8 |
| 89.0 | 12:88 | 6.4 | 47.8 | 19:81 | 0.7 | 3.1 |
| 91.0 | 11:89 | 3.8 | 29.2 | 17:83 | 0.7 | 3.4 |

| Second biotransformation (AD to ADD) | | | | | | |
|---|---|---|---|---|---|---|
| 0.0 | 11:89 | 3.8 | 29.2 | 17:83 | 0.7 | 3.4 |
| 18.0 | 53:47 | 23.2 | 20.7 | 70:30 | 3.1 | 1.3 |
| 22.0 | 77:23 | 18.9 | 5.4 | 89:11 | 3.0 | 0.4 |
| 26.0 | 79:21 | 34.3 | 9.1 | 89:11 | 7.6 | 1.0 |
| 41.5 | 75:25 | 9.3 | 3.0 | 86:14 | 6.8 | 1.1 |

**Table 8. Summary of the monitoring of Two Stage Biotarnsformation Process.**

| Time | Foam layer | | | Water layer | | |
|---|---|---|---|---|---|---|
| | ADD/AD | ADD | AD | ADD/AD | ADD | AD |
| (hrs) | (-) | (%) | (%) | (-) | (%) | (%) |
| First biotransformation (phytosterols to AD) | | | | | | |
| 0.0 | 0:0 | 0.0 | 0.0 | 0: 0 | 0.0 | 0.0 |
| 43.0 | 16:84 | 3.9 | 19.9 | 37:63 | 0.5 | 0.8 |
| 62.5 | 17:83 | 2.8 | 13.7 | 31:69 | 0.7 | 1.5 |
| 69.5 | 10:90 | 3.0 | 27.1 | 27:73 | 0.7 | 1.9 |
| 86.5 | 9:91 | 5.5 | 57.6 | 25:75 | 0.6 | 1.8 |
| 90.0 | 8:92 | 3.9 | 44.7 | 25:75 | 0.5 | 1.5 |

| Second biotransformation (AD to ADD) | | | | | | |
|---|---|---|---|---|---|---|
| 0.0 | 8:92 | 3.9 | 44.7 | 25 : 75 | 0.5 | 1.5 |
| 29.0 | 97:3 | 23.7 | 0.8 | 95: 5 | 4.3 | 0.2 |
| 46.0 | 94:6 | 19.6 | 1.3 | 95: 5 | 3.2 | 0.2 |

### EXAMPLE 5 IDENTIFICATION OF NEW MUTANT F. SOLANI FMI 33

Protocol was used to generate large subunit (LSU)rRNA gene sequence data. Approximately 300 base pairs of the LSU rRNAgene starting at position 3344 was PCR amplified from genomic D NA i solated form the F MI 33 fungal colonies. Amplication products were purified from excess primers and dNTPs using Microcon 100 (Amicon) molecular weight cut-off membranes and checked for quality and quantity by rinning a portion of the products on an agarose gel.

Cycle sequencing of the LSU rRNA amplification products was carried out using AmpliTaq FS DNA polymerase and dRhodamine dye terminators. Excess dye-labeled terminators were removed from the sequencing reactions using s Sephadex G-50 spin column. The products were collected by centrifugation, dried under vacuum and frozen at -20°C until ready to load. Samples were resuspended in a solution of formamide/blue dextran/EDTA and denatured prior to loading. The samples were electrophoresed on a ABI Prism 377 DNA sequencer. Data was analyzed using PE/Applied Biosystems DNA Editing and assembly software.

Comparisons were made between sample FMI 33 and other microorganisms in MicroSeq database using microbial analysis software. FMI 33 was found to have a genetic distance from the next closest match, *F. so*/*ani,* of 0.94%, which is indicative of a separate fungal mutant.

Sequence Data of FMI 33:

AGACCGATAGCGAACAAGTAGAGTGATCGAAAGATGAAAAGAACTTTGAAAAGAG
AGTTA

AACAGTACGTGAAATTGTTGAAAGGGAAGCGCTTGTGACCAGACTTGGGCTTGGTT
GATC

ATCCGGGGTTCTCCCCGGTGCACTCTTCCGGCCAGGCCAGCATCAGTTCGCCCTGGG
GG

ACAAAGGCATCGGGAACGTGGCTCTCTCCGGGGAGTGTTATAGCCCGTTGCGTAAT
ACCC

TGCGGCGGACTGAGGTTCGCGCATTCGCAAGGATGCTGGCGTAATGGTCATCAGTG
ACCC

GTCTTGAAACACGGACCAAG

## Claims

1. A process of fermenting a phytosterol composition to produce (androst-4-ene-3,17-dione) and subsequently (androsta-1,4-diene-3,17-dione) which comprises:
propagating a microbial culture of the genus Mycobacterium in a first nutrient medium;
placing the microbial culture and the phytosterol composition in a bioreactor for a sufficient time to transform the composition substantially to androst-4-ene-3,17-dione ;
propagating a fungal culture of the genus Fusarium in a second nutrient medium;
injecting one or more of 1) Fusarium sp; and 2) the fungal culture medium into the bioreactor for a sufficient time to transform the and rost-4-ene-3,17-dione to androsta-1,4 diene-3, 17-dione.

2. The process of claim 1 wherein the fungal culture is Fusarium solani.

3. The process of claim 1 wherein the fungal culture is Fusarium oxysporum.

4. The process of claim 1 wherein the microbial culture is Mycobacterium MB 3683, deposited at ATCC as PTA-352.

5. The process of claim 1 wherein the fungal culture is Fusarium solani FMI 33, deposited at ATCC as PTA-3947.

6. The process of claim 1 wherein the phytosterol composition is derived from tall oil pitch or pulping soap.

7. The process of claim 1 wherein the phytosterol composition is derived from a suitable selected vegetable oil.

8. The process of claim 7 wherein the vegetable oil is selected from the group comprising soy, rapeseed, corn, cottonseed, sunflower, olive, linseed, or rice bran.

9. The process of claim 1 wherein the phytosterol composition comprises one or more of the following: sitosterol, campesterol, stigmasterol, brassicasterol, desmosterol, chalinosterol, poriferasterol, clionasterol, all hydrogenated counterparts and all natural or synthesized forms and derivatives thereof, including isomers.

10. The process of claim 1 wherein the first nutrient medium comprises Refiners molasses and inorganic salts.

11. The process of claim 1 wherein the second nutrient medium comprises glucose and corn steep liquor.

12. The process of claim 1 wherein the fermentations are carried out aerobically.

13. The process of claim 1 wherein the phytosterol composition is dissolved into a solution using a solubilizing agent and then added to the bioreactor.

14. The process of claim 13 wherein the solubilizing agent is a member of the glycol family.

15. The process of claim 13 wherein the solubilizing agent is polypropylene glycol.

16. The process of claim 13 wherein the solubilizing agent is a member of the silicone family.

17. The process of claim 1 wherein the bioreactor is sterilized and then cooled prior to the injection of 1) the fungal culture or 2) the fungal culture medium.

18. Fusarium solani FMI 33, deposited at ATCC as PTA-3947.

## Patentansprüche

1. Verfahren zum Fermentieren einer Phytosterol-Zusammensetzung, um ein Androst-4-en-3,17-dion und nachfolgend Androsta-1,4-dien-3,17-dion zu erzeugen, welches umfasst:
Propagieren einer mikrobiellen Kultur des Gattung Mykobakterium in einem ersten Nährmedium;
Anordnung der mikrobiellen Kultur und der Phytosterol-Zusammensetzung in einem Bioreaktor für eine ausreichende Zeit, um die Zusammensetzung im Wesentlichen in Androst-4-en-3,17-dion umzuwandeln;
Propagieren einer Pilzkultur des Gattung Fusarium in einem zweiten Nährmedium;
Injizieren von einer oder mehreren von 1) Fusarium SP und 2) des Pilzkultur-Mediums in dem Bioreaktor fiir eine ausreichende Zeit, um das Androst-4-en-3,17-dion in Andrasta-1,4-dien 3,17-dion umzuwandeln.

2. Verfahren gemäss Anspruch 1, bei dem die Pilzkultur Fusarium solani ist.

3. Verfahren gemäss Anspruch 1, bei dem die Pilzkultur Fusarium oxysporum ist

4. Verfahren gemäss Anspruch 1, bei dem die mikrobielle Kultur Mykobakterium MB3683 ist, hinterlegt bei der ATCC als PTA-352.

5. Verfahren gemäss Anspruch 1, bei dem die Pilzkultur Fusarium Solani FMI33 ist, hinterlegt bei der ATCC als PTA-3947.

6. Verfahren gemäss Anspruch 1, bei dem die Phytosterol-Zusammensetzung von Tallölpech oder Aufschlussseife abgeleitet ist

7. Verfahren gemäss Anspruch 1, bei dem die Phytosterol-Zusammensetzung von einem geeignet ausgewählten pflanzlichen Öl abgeleitet ist.

8. Verfahren gemäss Anspruch 7, bei dem das pflanzliche Öl ausgewählt ist aus der Gruppe umfassend Soja, Raps, Mais, Baumwollsamen, Sonnenblumen, Oliven, Leinsamen oder Reiskleie.

9. Verfahren gemäss Anspruch 1, bei dem die Phytosterol-Zusammensetzung eines oder mehrere der folgenden umfasst: Sitosterol, Campesterol, Stigmasterol, Brassicasterol, Desmosterol, Chalinosterol, Poriferasterol, Clionasterol, alle hydrierten Pendants und alle natürlichen oder synthetisierten Formen und Derivate davon, einschliessend die Isomere.

10. Verfahren gemäss Anspruch 1, bei dem das erste Nährmedium Raffineriemelasse und anorganische Salze umfasst.

11. Verfahren gemäss Anspruch 1, bei dem das zweite Nährmedium Glukose und Maisquellwasser umfasst.

12. Verfahren gemäss Anspruch 1, bei dem die Fermentationen aerob ausgeführt werden.

13. Verfahren gemäss Anspruch 1 bei dem die Phytosterol-Zusammensetzung in einer Lösung aufgelöst wird, die einen Lösungsvermittler enthält, und dann zu dem Bioreaktor hinzugefügt wird.

14. Verfahren gemäss Anspruch 13, bei dem der Lösungsvermittler ein Mittel der Glykolfamilie ist.

15. Verfahren gemäss Anspruch 13, bei dem der Lösungsvermittler Polypropylenglykol ist

16. Verfahren gemäss Anspruch 13, bei dem der Lösungsvermittler ein Mitglied der Silikonfamilie ist.

17. Verfahren gemäss Anspruch 1, bei dem der Bioreaktor sterilisiert wird und dann abgekühlt wird, vor der Injektion von 1) der Pilzkultur oder 2) des Pilzkulturmediums.

18. Fusarium solani FMI-33, hinterlegt bei der ATCC als PTA-3947.

## Revendications

1. Un procédé de fermentation d'une composition de phytostérol pour produire de l'androst-4-éne-3,17-dione et ensuite de l'androsta-1,4-diène-3,17-dione qui comprend :
la propagation d'une culture microbienne du genre Mycobacterium dans un premier milieu nutritif ;
le chargement de la culture microbienne et de la composition de phytostérol dans un bioréacteur pendant une durée de temps suffisante pour transformer en grande partie la composition en androst-4-éne-3,17-dione ;
la propagation d'une culture fongique du genre Fusarium dans un second milieu nutritif ;
l'injection d'un ou plusieurs des esp. Fusarium 1); et du milieu de culture fongique 2) dans le bioréacteur pendant une durée de temps suffisante pour transformer l'androst-4-éne-3, 17-dione en androsta-1,4-diène-3,17-dione.

2. Le procédé selon la revendication 1, dans lequel la culture fongique est celle de Fusarium solani.

3. Le procédé selon la revendication 1, dans lequel la culture fongique est celle de Fusarium oxysporum.

4. Le procédé selon la revendication 1, dans lequel la culture microbienne est celle de Mycobacterium MB 3683, déposée auprès de l'ATCC sous le numéro PTA-352.

5. Le procédé selon la revendication 1, dans lequel la culture fongique est celle de Fusarium solani FMI 33, déposée auprès de l'ATCC sous le numéro PTA-3947.

6. Le procédé selon la revendication 1, dans lequel la composition de phytostérol dérive de brai de tallol ou de pâte de savon.

7. Le procédé selon la revendication 1, dans lequel la composition de phytostérol dérive d'une huile végétale sélectionnée appropriée.

8. Le procédé selon la revendication 7, dans lequel l'huile végétale est choisie dans le groupe comprenant les huiles de soja, colza, maïs, graine de coton, tournesol, olive, graine de lin, ou son de riz.

9. Le procédé selon la revendication 1, dans lequel la composition de phytostérol comprend un ou plusieurs des composés suivants : sitostérol, campestérol, stigmastérol, brassicastérol, desmostérol, chalinostérol, poriférastérol, clionastérol, toutes les contreparties hydrogénées et toutes les formes naturelles ou synthétiques et dérivés de celles-ci, incluant les isomères.

10. Le procédé selon la revendication 1, dans lequel le premier milieu nutritif comprend des molasses de raffineurs et des sels inorganiques.

11. Le procédé selon la revendication 1, dans lequel le second milieu nutritif comprend du glucose et de la liqueur de maïs.

12. Le procédé selon la revendication 1, dans lequel les fermentations sont réalisées en aérobiose.

13. Le procédé selon la revendication 1, dans lequel la composition de phytostérol est dissoute dans une solution à l'aide d'un agent solubilisant, et est ensuite ajoutée dans le bioréacteur.

14. Le procédé selon la revendication 13, dans lequel l'agent solubilisant est un membre de la famille des glycols.

15. Le procédé selon la revendication 13, dans lequel l'agent solubilisant est du polypropylène glycol.

16. Le procédé selon la revendication 13, dans lequel l'agent solubilisant est un membre de la famille des silicones.

17. Le procédé selon la revendication 1, dans lequel le bioréacteur est stérilisé et est ensuite refroidi préalablement à l'injection de la culture fongique 1) ou du milieu de culture fongique 2).

18. Fusarium solani FMI 33, déposé auprès de l'ATCC sous le numéro PTA-3947.
